# EUROPEAN PATENT SPECIFICATION

(11) **EP 1 641 773 B1**
(45) Date of publication and mention of the grant of the patent: **23.07.2008**
(21) Application number: 04741907.2
(22) Date of filing: 28.06.2004
(51) Int. Cl.: C07D 333/56

(54) **PROCESS FOR PREPARING RALOXIFENE HYDROCHLORIDE**
VERFAHREN ZUR HERSTELLUNG VON RALOXIFENE-HYDROCHLORID
PROCEDE POUR PREPARER DE L'HYDROCHLORURE DE RALOXIFENE

(30) Priority: 30.06.2003 IT MI20031333
(43) Date of publication of application: 05.04.2006
(73) Proprietor: ERREGIERRE S.p.A., 24060 San Paolo D'Argon (Bergamo) (IT)
(72) Inventor: FERRARI, Massimo, I-24069 CENATE SOTTO (IT); ZINETTI, Fabrizio, I-24060 CASAZZA (IT); BELOTTI, Paolo, I-24060 SAN PAOLO D'ARGON (IT)
(74) Representative: Gervasi, Gemma
(86) International application number: PCT/EP2004/051263
(87) International publication number: WO 2005/003116

(56) References cited:
- EP-A- 0 062 503
- US-A- 4 418 068

## Description

### Field of the invention

The present invention relates to a process for preparing raloxifene and in particular high purity raloxifene hydrochloride with high yields.

### State of the art

Raloxifene and in particular the relating hydrochloride salt, characterised by the following formula (I): 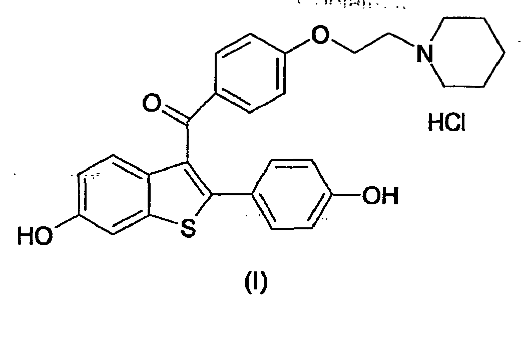 is an active principle used in the treatment of osteoporosis and was described for the first time in European patent application EP62503. In this prior patent various preparation methods are described which generally involve the following stages:
1) protection of the 2 hydroxylic functions of 6-hydroxy-2-(4-hydroxyphenyl)benzo[b]thiophene according to the following reaction scheme 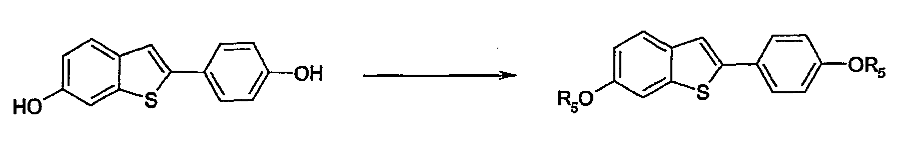 where R₅ is an alkyl, cycloalkyl or COR₆ acyl group, a SO₂R₆ sulfonyl group where R₆ is a primary or secondary C₁-C₄ alkyl, C₁-C₃ fluoro alkyl or C₁-C₄ alkoxyphenyl,
2) acylation of the compound protected with 4-(2-piperidinoethoxy)benzoyl halide according to the following synthesis scheme: 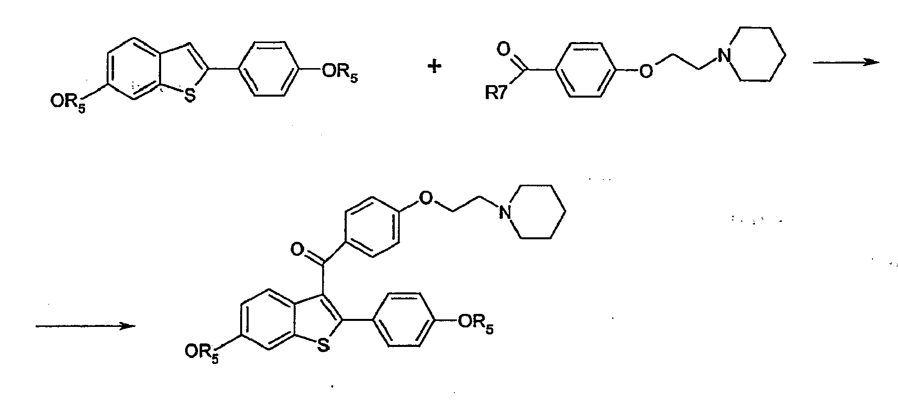 in which R₇ is a halogen atom,
3) deprotection or elimination of the OR₅ protective group.

As it results from the examples reported in EP62503, when the reaction is conducted using the acetyloxy group as OR₅ protective group, deprotection of this group is conducted first with sodium hydroxide in an alcoholic solution and subsequently with methanesulfonic acid. This type of hydrolysis however does not allow high purity raloxifene to be obtained, since, as indicated by example 6, the product to be purified must be passed through a chromatographic column. This type of treatment, however, only enables a yellow foam to be obtained, and, to arrive at a product of solid crystalline form, a further treatment with acetone is required. The crystallized product thus obtained consisting of raloxifene methanesulfonate must be further converted into the corresponding hydrochloride for pharmaceutical use.

The aforesaid process, requiring product passage through a chromatographic column, is not achievable at industrial level, proof of which being that in the same prior patent, instead of the aforesaid synthesis scheme, the one preferred is that in which the OR₅ protective group is an alkoxy, specifically a methoxy group, which for unblocking requires the use of aluminium trichloride and a thioderivative and preferably methanethiol, moreover in a quantity greatly in excess of the substrate on which the deprotection must be conducted, with considerable pollution problems, which evidently involves the use of considerable quantities of thioderivatives.

The processes described in EP62503 involve another inconvenience caused by the use of aluminium trichloride and, if proceeding to the scheme preferred by this prior patent, this Lewis acid must be used in substantial quantities, since it is used not only in stage (2) of acylation, but also in subsequent dealkylation. Aluminium trichloride as shown in the subsequent patent US5629425 produces a large quantity of aluminium-based by-products which are soluble in raloxifene processing solvents and are found therefore in the final product.

To overcome these problems, in the aforestated US5629425 boron trichloride or boron tribromide is used as Lewis acid, these being decidedly more expensive catalysts than aluminium trichloride.

The need was felt to provide a process which enabled raloxifene hydrochloride to be prepared with high yields and high purity and low aluminium content without using expensive catalysts.

### Summary of the invention

The applicant has surprisingly found a process capable of overcoming the drawbacks of known processes and which allows raloxifene and in particular raloxifene hydrochloride to be obtained with high purity and high yields.

This process comprises in particular the following stages:
a) demethylation of 6-methoxy-2-(4-methoxyphenyl)benzo[b]thiophene of formula (II) 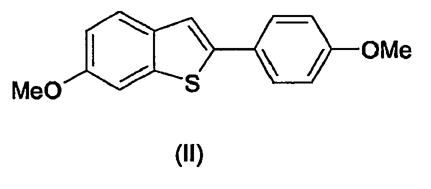 in pyridine hydrochloride to obtain 6-hydroxy-2-(4-hydroxyphenyl)benzo[b]thiophene of formula (III) 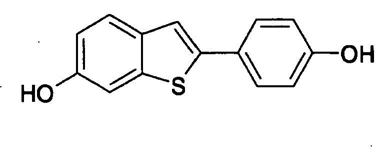
b) acetylation of 6-hydroxy-2-(4-hydroxyphenyl)benzo[b]thiophene with an acetylating agent to obtain the corresponding 6-acetoxy-2-(4-acetoxyphenyl)benzo[b]thiophene of formula (IV) 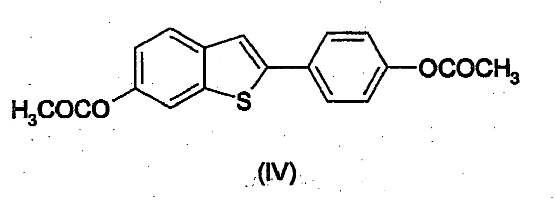
c) acylation of 6-acetoxy-2-(4-acetoxyphenyl)benzo[b]thiophene (IV) with 4-(2-piperidinoethoxy)benzoylchloride hydrochloride of formula (V) 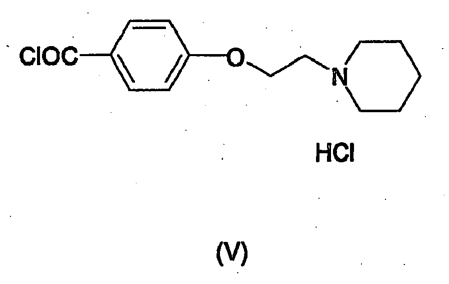 with aluminium trichloride in halogenated solvent to obtain 6-acetoxy-2-(4 acetoxyphenyl)-3-[4-(2-piperidinoethoxy)benzoyl]-benzo[b]thiophene of formula (VI) 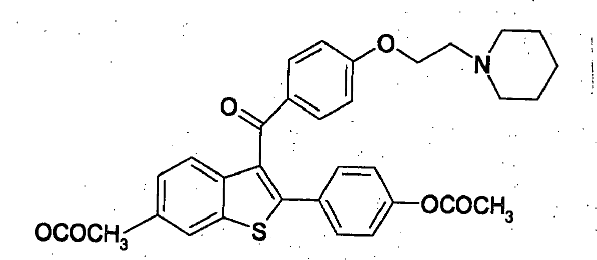
d) hydrolysis of 6-acetoxy-2-(4-acetoxyphenyl)-3-[4-(2-piperidinoethoxy)benzoyl]-benzo[b]thiophene, according to the following operative methods:
   d1) treatment of 6-acetoxy-2-(4-acetoxyphenyl)-3-[4-(2-piperidinoethoxy)benzoyl]-benzo[b]thiophene with alkaline hydroxide in alcohol solvent,
   d2) acidification of the product obtained in the previous stage (d1) with a strong acid, to obtain the corresponding raloxifene salt with strong acid, **characterised in that** :
      - stage (d1) is conducted using methanol as alcohol solvent and excess 30% sodium hydroxide,
      - the strong acid used in stage (d2) is concentrated hydrochloric acid and said stage (d2) is conducted directly on the reaction mixture derived from stage (d1), to which equal weight quantities of water and ethyl acetate and finally 37% concentrated hydrochloric acid are added,
      - the suspension obtained in stage (d2) is washed with equal weight quantities of water and ethyl acetate.

In this respect, by conducting the hydrolysis of 6-acetoxy-2-(4-acetoxyphenyl)-3-[4-(2-piperidinoethoxy)benzoyl]-benzo[b]thiophene with sodium hydroxide and subsequently treating the product obtained with hydrochloric acid in place of methanesulfonic acid, raloxifene hydrochloride precipitates in crystalline form directly with a high purity equal to 98%, thus in contrast to the analogous process described in EP65203 conducted with methanesulfonic acid, without having to use purification processes such as passage through a chromatographic column, which are impractical from the industrial point of view. In addition the product derived from stage (d2) has a low aluminium content.

### Detailed description of the invention

The 6-methoxy-2-(4-methoxyphenyl)benzo[b]thiophene of formula (II) used in stage (a) of the process of the present invention is prepared by reacting 3-methoxybenzene-thiol with α-bromo-4-methoxyacetophenone to obtain the corresponding α-(3-methoxyphenylthio)-4-methoxyacetophenone which is finally cyclizised to obtain the intermediate (II) with polyphosphoric acid, as in the following scheme.

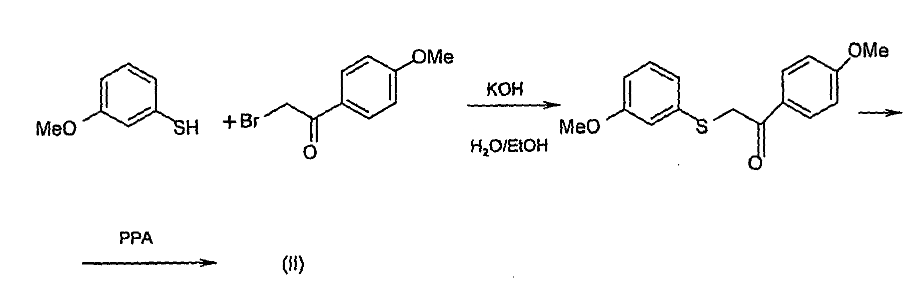

The pyridine hydrochloride used in stage (a) is preferably prepared in situ by adding concentrated hydrochloric acid to pyridine and distilling off all the water to obtain a thick but stirrable residue. The applicant has also surprisingly found that if the demethylation reaction or stage (a) of the process of the present invention is conducted in the presence not only of pyridine hydrochloride but also of tributylamine, preferably in weight ratios with respect to 6-methoxy-2-(4-methoxyphenyl)benzo[b]thiophene (II) of between 0.5 and 2, it is possible to lower the reaction temperature which in prior art is conducted at 210°-C, to decidedly lower temperatures, between 170 and 180°C.

According to a preferred embodiment of the process of the present invention, it is not necessary to isolate the 6-hydroxy-2-(4-hydroxyphenyl)benzo[b]thiophene (III) obtained in stage (a).

In stage (b) according to a preferred embodiment acetic anhydride is used as acetylating agent and a tertiary aliphatic amine, preferably triethylamine, is used as hydrogen ion acceptor. The solvent used in stage (a) is an aprotic polar solvent, ethyl acetate being particularly preferred.

The 4-(2-piperidinoethoxy)benzoylchloride hydrochloride of formula (V) used in stage (c) is preferably prepared in situ by a conventional type procedure by reacting 4-(2-piperidinoethoxy)-benzoic acid hydrochloride with thionyl chloride without isolating the reaction product. This reaction is preferably conducted in methylene chloride in the presence of pyridine as catalyst.

Stage (c) is preferably conducted in methylene chloride, according to a particularly preferred embodiment this stage being conducted in the following manner: 6-acetoxy-2-(4-acetoxyphenyl)benzo[b]thiophene is added to 4-(2-piperidinoethoxy)benzoylchloride hydrochloride of formula (V) prepared in situ while still in its reaction solvent methylene chloride, the mixture thus obtained being poured onto a mixture consisting of methylene chloride and aluminium trichloride.

According to a preferred embodiment of the process of the present invention, 6-acetoxy-2-(4-acetoxyphenyl)-3-[4-(2-piperidinoethoxy)benzoyl]-benzo[b]thiophene (VI) is not isolated but is used in crude form for the subsequent hydrolysis (d).

Stage (d1) is conducted using methanol as the alcoholic solvent, with excess 30% sodium hydroxide.

Stage (d2) is conducted directly on the reaction mixture derived from stage (d1) to which equal weight quantities of water and ethyl acetate are added and finally 37% concentrated hydrochloric acid.

A suspension is hence obtained, which is washed with equal weight quantities of water and ethyl acetate.

By the process of the present invention raloxifene hydrochloride is obtained with high purity and high yields of about 65-70% calculated on the 6-methoxy-2-(4-methoxyphenyl)benzo[b]thiophene (II).

The applicant has also found that if raloxifene hydrochloride obtained by the process of the present invention is crystallised from an alcoholic solvent, preferably methanol, possibly in the presence of small quantities of HCI, it achieves a purity of greater than 99%.

Finally the applicant has also found that by conducting a further crystallization, again from an alcoholic solvent, preferably methanol, possibly in the presence of HCI, on the product derived from the first crystallisation, raloxifene hydrochloride can be obtained with a purity greater than 99.7%. In particular raloxifene hydrochloride obtained after the first and/or the second crystallisation contains the characteristic impurity consisting of raloxifene hydrochloride N-oxide in a quantity less than 0.05% and preferably less than 0.01%, this product also having an aluminium content less than 5 ppm.

The product thus obtained has a particle size distribution (after gentle grinding conducted with the aim of simply homogenising the product) such that D(0.9) is ≤100µm and D(0.5) ≥40µm. By further sieving a raloxifene hydrochloride is obtained with the following particle size distribution: D(0.9) between 50 and 65µm and D[4.3] ≥20µm.

Some illustrative but non-limiting examples of the preparation process for raloxifene hydrochloride of the present invention and its relative intermediates are given.

### EXAMPLE 1

### Preparation of 6-acetoxy-2-(4-acetoxyphenyl)benzo[b]thiophene (IV).

24 kg of pyridine (0.303 kmol) and 28.8 kg of 37% hydrochloric acid (0.292 kmol) are fed into a reactor. The reactor is placed under vacuum and all the water is distilled off until a thick but stirrable residue is obtained.

The residue is then redissolved in 6 kg of tributylamine and 6 kg of 6-methoxy-2-(4-methoxyphenyl)benzo[b]thiophene (0.022 kmol). The mixture is heated to 170-180°C and is maintained at this temperature for some hours. It is then cooled to 50-60°C and 24 kg of ethyl acetate and 60 kg of deionised water are fed into the reactor. The mixture is stirred for 15 minutes and the phases are separated. The solvent is distilled off from the organic phase under vacuum and the residue is redissolved with 24 kg of ethyl acetate and 5.3 kg of triethylamine (0.052 kmol).

The mixture obtained is heated to 60-65°C while being stirred and 8.9 kg of acetic anhydride (0.087 kmol) are added. The reaction mixture is stirred for 1 hour at the same temperature then is cooled to 25-30°C and 24 kg of deionised water are added. The suspension is centrifuged, washed with 6 kg of deionised water and 6 kg of ethyl acetate.

The product is then dried at 50-60°C and about 6.6 kg of dried product are obtained. The reaction yield is 91.1%.

### EXAMPLE 2

### Preparation of crude raloxifene hydrochloride.

### PHASE A

42 kg of methylene chloride and 7.8 kg of 4-(2-piperidinoethoxy)-benzoic acid hydrochloride (0.027 kmol), 0.12 kg pyridine (0.0015 kmol) are fed into a reactor and heated under reflux and then 3.96 kg of thionyl chloride (0.033 kmol) are added. The mixture is stirred for 1 hour then about 20 litres of methylene chloride are distilled off. The mixture is cooled to 20-30°C and 6 kg of 6-acetoxy-2-(4-acetoxyphenyl)benzo[b]thiophene (IV) (0.018 kmol) are added.

The mixture is stirred until is completely homogenised.

### PHASE B

36 kg of methylene chloride and 16.8 kg of aluminium trichloride (0.126 kmol) are fed into a reactor.

While stirring, the chloromethylene suspension, comprised of phase A prepared as described above, is added at 15-30°C. The mixture is stirred for 1 hour then the entire reaction mixture is poured into a reactor containing 60 kg of ice.

The mixture is stirred at 15-30°C then the suspension is centrifuged, washing with 3 kg of methylene chloride and 3 kg of deionised water.

The centrifuged mother liquors, containing the product, are fed into a reactor and the phases are separated. The organic phase is distilled off until obtaining an oily residue and 15 kg of methyl alcohol are added, stirred at 20-40°C and, maintaining the same temperature, 9.1 kg of 30% sodium hydroxide (0.068 kmol) are poured in. The mixture is stirred for 1 hour and 30 kg of deionised water and 30 kg of ethyl acetate are added.

At the same temperature 7.2 kg of 37% hydrochloric acid (0.073 kmol) are then added. The suspension is centrifuged, washing with 6 kg of ethyl acetate and 6 kg of deionised water. At the end 6.6 kg of dried product with HPLC purity > 98% and low aluminium content are obtained. The reaction yield calculated on the 6-acetoxy-2-(4-acetoxyphenyl)benzo[b]thiophene (IV) is equal to a yield of 70.4%.

### EXAMPLE 3

### Crystallisation of crude raloxifene hydrochloride (1^{st} crystallisation of crude raloxifene hydrochloride)

6 kg of deionised water, 6 kg of crude raloxifene hydrochloride prepared as described in example 2 and 107 kg of methyl alcohol are fed into a reactor. The reaction mixture is heated until a complete solution is obtained then 0.25 kg of decolourising carbon are added. It is stirred for 15 minutes and then the suspension is filtered. While maintaining the solution stirred, 67 kg of methyl alcohol are distilled off. The residue is cooled and 0.1 kg of 37% hydrochloric acid are added. The pH, which must not exceed 2, is checked and the reaction mixture is then stirred for 2 hours at 20-40°C. The suspension is centrifuged, washing with 6 kg of methyl alcohol. 4.5 kg of dried product are obtained with HPLC purity of >99% and a yield of 75%.

### EXAMPLE 4

### Crystallisation of crystalline raloxifene (2^{nd} crystallisation).

0.9 kg of deionised water, 81 kg of methanol and the entire amount of crystallised product as described in example 3 are fed into a reactor. While maintaining the reaction mixture under stirring it is heated under reflux and 36 kg of methyl alcohol are distilled off. It is then cooled to 20-40°C and 0.08 kg of 37 % hydrochloric acid are added. The suspension is centrifuged, washing with 4 kg of methyl alcohol. The product is dried at 70°C. 4 kg of raloxifene hydrochloride are obtained with HPLC purity > 99.8%, reaction yield 89%, in particular the raloxifene hydrochloride N-oxide content is less than 0.01% and aluminium content is less than 5ppm. In particular the raloxifene hydrochlordie obtained after crystallisation contains the characteristic impurity consisting of raloxifene hydrochloride N-oxide in a quantity less than 0.05% and preferably less than 0.01%. The product thus obtained has a particle size distribution (after gentle grinding conducted with the aim of simply homogenising the product) such that D(0.9) is ≤100µm and D (0.5) ≥40µm.

By further sieving a raloxifene hydrochloride is obtained with the following particle size distribution: D(0.9) between 50 and 65µm and D[4.3] ≥20µm.

## Claims

1. Process for preparing raloxifene hydrochloride of formula (I) 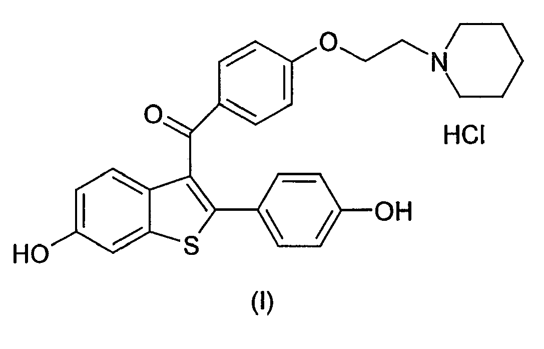 with a HPLC purity higher than 98% comprising the following stages:
a) demethylation of 6-methoxy-2-(4-methoxyphenyl)benzo[b]thiophene of formula (II) 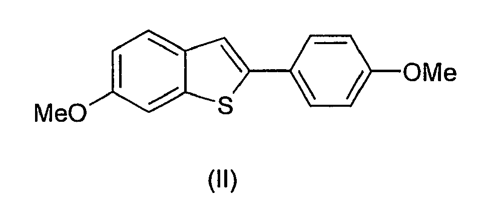 in pyridine hydrochloride to obtain 6-hydroxy-2-(4-hydroxyphenyl)benzo[b]thiophene of formula (III) 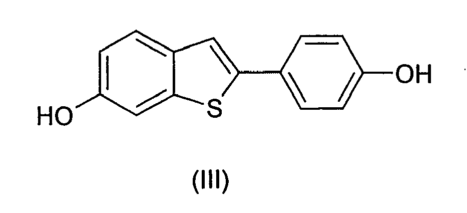
b) acetylation of 6-hydroxy-2-(4-hydroxyphenyl)benzo[b]thiophene with an acetylating agent to obtain the corresponding 6-acetoxy-2-(4-acetoxyphenyl)benzo[b]thiophene of formula (IV) 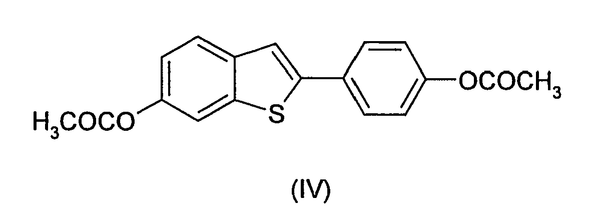
c) acylation of 6-acetoxy-2-(4-acetoxyphenyl)benzo[b]thiophene (IV) with 4-(2-piperidinoethoxy)benzoylchloride hydrochloride of formula (V) 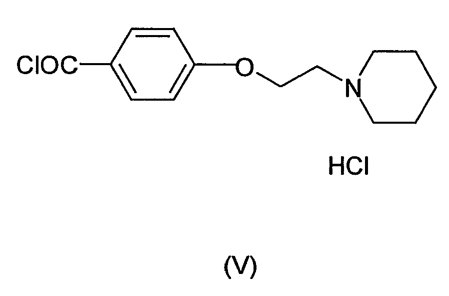 with aluminium chloride in halogenated solvent to obtain 6-acetoxy-2-(4-acetoxyphenyl)-3-[4-(2-piperidinoethoxy)benzoyl]-benzo[b]thiophene of formula (VI) 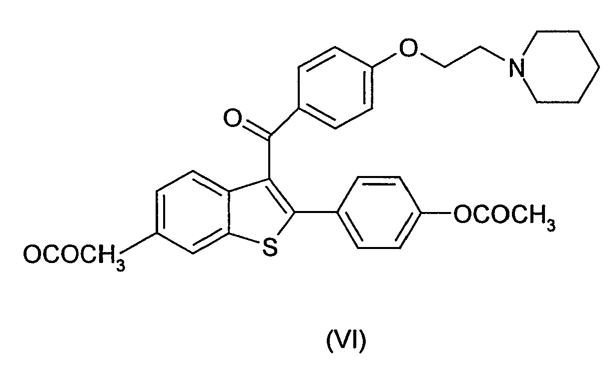
d) hydrolysis of 6-acetoxy-2-(4-acetoxyphenyl)-3-[4-(2-piperidinoethoxy)benzoyl]-benzo[b]thiophene, according to the following operative modalities:
d1) treatment of 6-acetoxy-2-(4-acetoxyphenyl)-3-[4-(2-piperidinoethoxy)benzoyl]-benzo[b]thiophene with alkaline hydroxide in alcohol solvent,
d2) acidification of the product obtained in the preceding stage (d1) with a strong acid, to obtain the corresponding raloxifene salt with the strong acid, **characterised in that**:
• stage (d1) is conducted using methanol as alcohol solvent and excess 30% sodium hydroxide,
• the strong acid used in stage (d2) is concentrated hydrochloric acid and said stage (d2) is conducted directly on the reaction mixture derived from stage (d1), to which equal weight quantities of water and ethyl acetate and finally 37% concentrated hydrochloric acid are added
• the suspension obtained in stage (d2) is washed with equal weight quantities of water and ethyl acetate.

2. Process as claimed in claim 1, **characterised in that** the pyridine hydrochloride used in stage (a) is prepared in situ by adding concentrated hydrochloric acid to pyridine and distilling off all the water to obtain a thick but stirrable residue.

3. Process as claimed in claim 1 or 2, **characterised in that** the demethylation reaction or stage (a) of the process of the present invention is also conducted in the presence or tributylamine.

4. Process as claimed in claim 3, **characterised in that** tributylamine is used in weight ratios with respect to 6-methoxy-2-(4-methoxyphenyl)benzo[b]thiophene (II) of between 0.5 and 2.

5. Process as claimed in claim 4, **characterised in that** stage (a) is conducted at a temperature between 170 and 180°C.

6. Process as claimed in any one of claims 1-5, **characterised in that** acetic anhydride is used as acetylating agent in the presence of triethylamine in ethyl acetate.

7. Process as claimed in any one of claims 1-6, **characterised in that** the 4-(2-piperidinoethoxy)benzoylchloride hydrochloride of formula (V) used in stage (c) is prepared in situ, by reacting 4-(2-piperidinoethoxy)benzoic acid hydrochloride with thionyl chloride in methylene chloride in the presence of pyridine, without isolating the reaction product.

8. Process as claimed in any one of claims 1-7, **characterised in that** stage (c) is conducted in methylene chloride.

9. Process as claimed in claim 8, **characterised in that** stage (c) is conducted according to the following operative modalities: 6-acetoxy-2-(4-acetoxyphenyl)benzo[b]thiophene (IV) is added to non-isolated 4-(2-piperidinoethoxy)benzoylchloride hydrochloride of formula (V) and prepared in situ as in claim 7 and the aforesaid mixture is poured onto a mixture consisting of methylene chloride and aluminium trichloride.

10. Process as claimed in any one of claims 1-9, **characterised in that** the 6-acetoxy-2-(4-acetoxyphenyl)benzo[b]thiophene (IV) is not isolated, but is used in the crude state in the subsequent reaction (d).

11. Process as claimed in any one of claims 1-10, **characterised in that** raloxifene hydrochloride derived from stage (d2) is crystallised from an alcoholic solvent.

12. Process as claimed in claim 11, **characterised in that** said solvent is methanol possibly in the presence of HCI.

13. Process as claimed in any one of claims 11 and 12, **characterised in that** raloxifene hydrochloride is obtained with a HPLC purity greater than 99%.

14. Process as claimed in any one of claims 12 and 13, **characterised in that** a further crystallisation from raloxifene hydrochloride from alcohol solvent is conducted.

15. Process as claimed in claim 14, **characterised in that** said crystallisation is conducted in methanol possibly in the presence of HCI.

16. Process according to claim 15 for preparing raloxifene hydrochloride with a HPLC purity greater than 99.7% and containing aluminium in a quantity less than 5 ppm %.

17. Process according to claim 16, **characterised in that it** contains raloxifene hydrochloride N-oxide in a quantity less than 0.05%.

18. Process according to claim 17, **characterised in that** said impurity is contained in a quantity less than 0.01 %.

19. Process according to claim 18, **characterised in that** raloxifene hydrochloride has a D(0.9) ≤100µm and a D(0.5) ≥40µm.

20. Process according to claim 19, **characterised** it comprises a further sieving, thereby obtaining a raloxifene hydrochloride having a D(0.9) between 50 and 65 µm and a D[4.3] ≥20µm.

## Patentansprüche

1. Verfahren zur Herstellung von Raloxifen-Hydrochlorid mit der Formel (I) 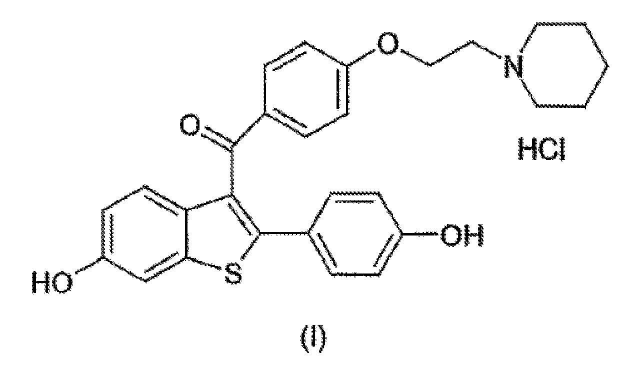 mit einer HPLC-Reinheit höher als 98 %, umfassend die folgenden Schritte:
a) Demethylierung von 6-Methoxy-2-(4-methoxyphenyl)benzo[b]thiophen der Formel (II) 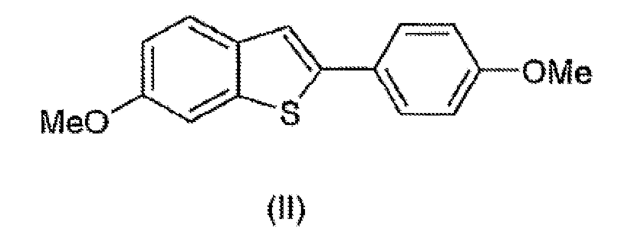 in Pyridin-Hydrochlorid, um 6-Hydroxy-2-(4-hydroxyphenyl)benzo[b]thiophen der folgenden Formel (III) zu erhalten 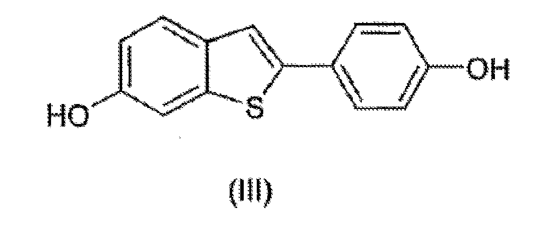
b) Acetylierung von 6-Hydroxy-2-(4-hydroxyphenyl)benzo[b]thiophen mit einem Acetylierungsmittel, um das entsprechende 6-Acetoxy-2-(4-acetoxyphenyl)benzo[b]thiophen der folgenden Formel (IV) zu erhalten 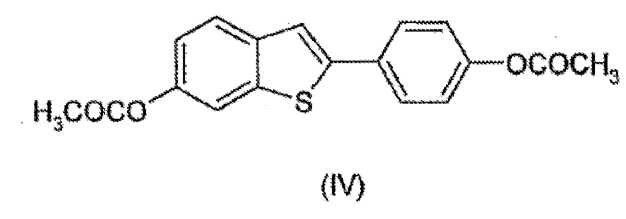
c) Acylierung von 6-Acetoxy-2-(4-acetoxyphenyl)benzo[b]thiophen (IV) mit 4-(2-Piperidinoethoxy)benzoylchlorid-Hydrochlorid der Formel (V) 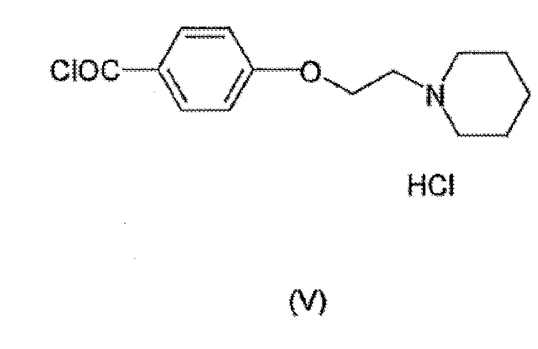 mit Aluminiumchlorid in halogeniertem Lösungsmittel, um 6-Acetoxy-2-(4-acetoxyphenyl)-3-[4-(2-piperidinoethoxy)benzoyl]benzo[b]thiophen der folgenden Formel (VI) zu erhalten 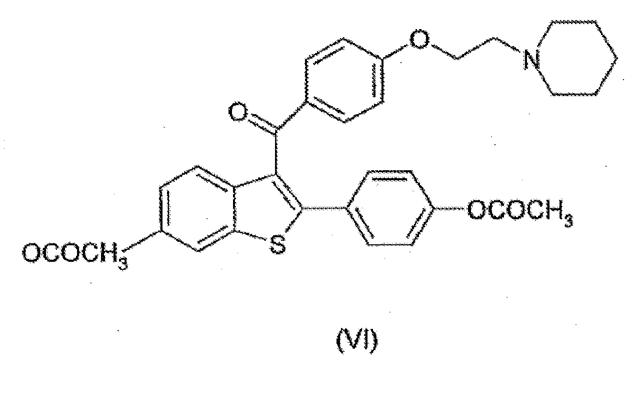
d) Hydrolyse von 6-Acetoxy-2-(4-acetoxyphenyl)-3-[4-(2-piperidinoethoxy)benzoyl]benzo[b]thiophen gemäß den folgenden Betriebsmodalitäten:
d1) Behandlung von 6-Acetoxy-2-(4-acetoxyphenyl)-3-[4-(2-piperidinoethoxy)benzoyl]benzo[b]thiophen mit einem alkalischen Hydroxid in einem alkoholischen Lösungsmittel,
d2) Ansäuerung des in der vorhergehenden Stufe (d1) erhaltenen Produkts mit einer starken Säure, um das entsprechende Raloxifensalz mit der starken Säure zu erhalten,
**dadurch gekennzeichnet, dass**:
• Stufe (d1) unter Verwendung von Methanol als alkoholisches Lösungsmittel und einem Überschuss an 30 %igem Natriumhydroxid ausgeführt wird,
• die in Stufe (d2) verwendete starke Säure konzentrierte Salzsäure ist und genannte Stufe (d2) direkt an der aus Stufe (d1) stammenden Reaktionsmischung durchgeführt wird, zu der gleiche Gewichtsmengen an Wasser und Ethylacetat und schließlich 37 %ige konzentrierte Salzsäure gegeben werden,
• die in Stufe (d2) erhaltene Suspension mit gleichen Gewichtsmenge an Wasser und Ethylacetat gewaschen wird.

2. Verfahren gemäß Anspruch 1, **dadurch gekennzeichnet, dass** das in Stufe (a) verwendete Pyridin-Hydrochlorid in situ durch Zugabe von konzentrierter Salzsäure zu Pyridin und Abdestillieren von Wasser hergestellt wird, um einen dicken, aber rührbaren Rückstand zu erhalten.

3. Verfahren gemäß Anspruch 1 oder 2, **dadurch gekennzeichnet, dass** die Demethylierungsreaktion oder Stufe (a) des Verfahrens der vorliegenden Erfindung auch in Gegenwart von Tributylamin durchgeführt wird.

4. Verfahren gemäß Anspruch 3, **dadurch gekennzeichnet, dass** Tributylamin in Gewichtsverhältnissen bezogen auf 6-Methoxy-2-(4-methoxyphenyl)benzo[b]thiophen (II) zwischen 0,5 und 2 verwendet wird.

5. Verfahren gemäß Anspruch 4, **dadurch gekennzeichnet, dass** diese Stufe (a) bei einer Temperatur zwischen 170 und 180 °C durchgeführt wird.

6. Verfahren gemäß einem der Ansprüche 1 - 5, **dadurch gekennzeichnet, dass** Essigsäureanhydrid als Acetylierungsmittel in Gegenwart von Triethylamin in Ethylacetat verwendet wird.

7. Verfahren gemäß einem der Ansprüche 1 - 6, **dadurch gekennzeichnet, dass** das in Stufe (c) verwendete 4-(2-Piperidinoethoxy)benzoylchlorid-Hydrochlorid der Formel (V) in Situ mittels Reaktion von 4-(2-Piperidinoethoxy)benzoesäure-Hydrochlorid mit Thionylchlorid in Methylenchlorid in Gegenwart von Pyridin ohne Isolierung des Reaktionsprodukts hergestellt wird.

8. Verfahren gemäß einem der Ansprüche 1 - 7, **dadurch gekennzeichnet, dass** Stufe (c) in Methylenchlorid durchgeführt wird.

9. Verfahren gemäß Anspruch 8, **dadurch gekennzeichnet, dass** Stufe (c) gemäß den folgenden Betriebsmodalitäten durchgeführt wird: 6-Acetoxy-2-(4-acetoxyphenyl)benzo[b]thiophen (IV) wird zum nicht isolierten und gemäß Anspruch 7 in Situ hergestellten 4-(2-Piperidinoethoxy)benzoylchlorid-Hydrochlorid der Formel (V) gegeben und die vorgenannte Mischung wird auf eine Mischung bestehend aus Methylenchlorid und Aluminiumtrichlorid gegossen.

10. Verfahren gemäß einem der Ansprüche 1 - 9, **dadurch gekennzeichnet, dass** das 6-Acetoxy-2-(4-acetoxyphenyl)benzo[b]thiophen (IV) nicht isoliert ist, sondern im rohen Zustand in der nachfolgenden Reaktion (d) verwendet wird.

11. Verfahren nach einem der Ansprüche 1 - 10, **dadurch gekennzeichnet, dass** aus Stufe (d2) stammendes Raloxifen-Hydrochlorid aus einem alkoholischen Lösungsmittel kristallisiert wird.

12. Verfahren gemäß Anspruch 11, **dadurch gekennzeichnet, dass** genanntes Lösungsmittel Methanol, gegebenenfalls in der Gegenwart von HCI, ist.

13. Verfahren gemäß einem der Ansprüche 11 und 12, **dadurch gekennzeichnet, dass** Raloxifen-Hydrochlorid mit einer HPLC-Reinheit von mehr als 99 % erhalten wird.

14. Verfahren gemäß einem der Ansprüche 12 und 13, **dadurch gekennzeichnet, dass** eine weitere Kristallisierung von Raloxifen-Hydrochlorid aus einem alkoholischen Lösungsmittel durchgeführt wird.

15. Verfahren gemäß Anspruch 14, **dadurch gekennzeichnet, dass** genannte Kristallisierung in Methanol, gegebenenfalls in der Gegenwart von HCI, durchgeführt wird.

16. Verfahren gemäß Anspruch 15 zur Herstellung von Raloxifen-Hydrochlorid mit einer HPLC-Reinheit von mehr als 99,7 %, das Aluminium in einer Menge von weniger als 5 ppm % enthält.

17. Verfahren gemäß Anspruch 16, **dadurch gekennzeichnet, dass** es Raloxifen-Hydrochlorid-N-oxid in einer Menge von weniger als 0,05 % enthält.

18. Verfahren gemäß Anspruch 17, **dadurch gekennzeichnet, dass** genannte Verunreinigung in einer Menge von weniger als 0,01 % enthalten ist.

19. Verfahren gemäß Anspruch 18, **dadurch gekennzeichnet, dass** Raloxifen-Hydrochlorid ein D(0,9) ≤ 100 µm und ein D(0,5) ≥ 40 µm aufweist.

20. Verfahren gemäß Anspruch 19, **dadurch gekennzeichnet, dass** es einen weiteren Siebvorgang umfasst, wodurch ein Raloxifen-Hydrochlorid mit einem D(0,9) zwischen 50 und 65 µm und einem D[4,3] ≥ 20 µm erhalten wird.

## Revendications

1. Procédé pour la préparation de chlorhydrate de raloxifène de la formule (I) 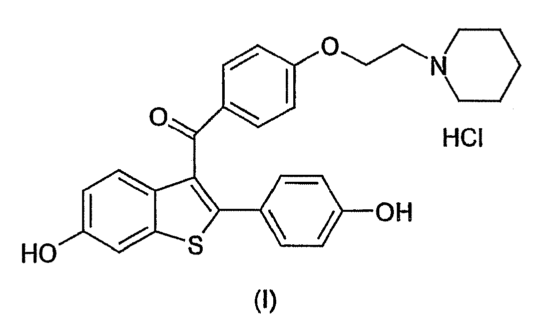 avec une pureté HPLC de plus de 98% comprenant les étapes suivantes :
a) déméthylation du 6-méthoxy-2-(4-méthoxyphényl) benzo [b]thiophène de la formule (II) 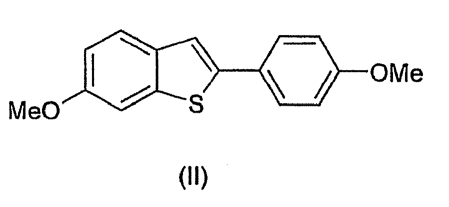 dans du chlorhydrate de pyridine pour obtenir du 6-hydroxy-2-(4-hydroxyphényl)benzo[b]thiophène de la formule (III) 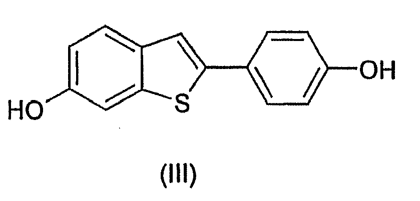
b) acétylation du 6-hydroxy-2-(4-hydroxyphényl) benzo [b]thiophène avec un agent acétylant pour obtenir le 6-acétoxy-2-(4-acétoxyphényl)benzo[b]thiophène correspondant de formule (IV) 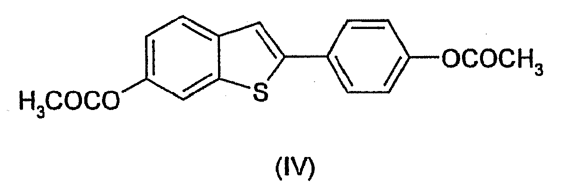
c) acylation du 6-acétoxy-2-(4-acétoxyphényl)benzo [b]thiophène (IV) avec du chlorhydrate de chlorure de 4-(2-pipéridinoéthoxy)benzoyle de formule (V) 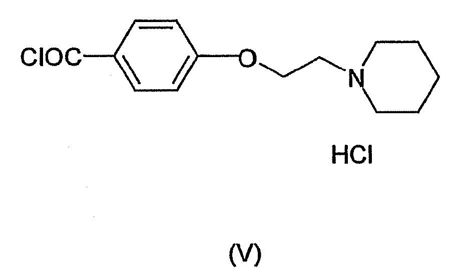 avec du chlorure d'aluminium dans un solvant halogéné pour obtenir du 6-acétoxy-2-(4-acétoxyphényl)-3-[4-(2-pipéridino-éthoxy)benzoyle]benzo[b]thiophène de formule (VI) 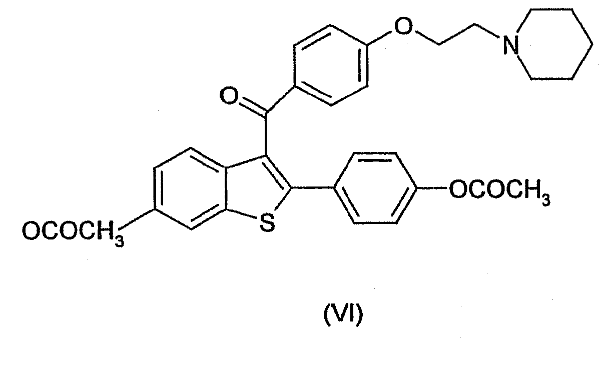
d) hydrolyse du 6-acétoxy-2-(4-acétoxyphényl)-3-[4-(2-pipéridinoéthoxy)benzoyl]-benzo[b]thiophène, selon les modalités opératives qui suivant :
d1) traitement du 6-acétoxy-2-(4-acétoxyphényl)-3-[4-(2-pipéridinoéthoxy)benzoyl]-benzo[b]thiophène avec un hydroxyde alcalin dans un alcool solvant,
d2) acidification du produit obtenu au stade (d1) précédent avec un acide fort pour obtenir le sel de raloxifène correspondant avec l'acide fort **caractérisé en ce que**
• le stade (d1) est entrepris en utilisant du méthanol comme alcool solvant et de l'hydroxyde de sodium en excès de 30%,
• l'acide fort utilisé au stade (d2) est de l'acide chlorhydrique concentré et ledit stade (d2) est entrepris directement sur le mélange réactionnel dérivé du stade (d1), auquel des quantités de poids égales d'eau et d'acétate d'éthyle et enfin de l'acide chlorhydrique concentré à 37% sont ajoutés
• la suspension obtenue au stade (d2) est lavée avec des quantités égales en poids d'eau et d'acétate d'éthyle.

2. Procédé selon la revendication 1 **caractérisé en ce que** le chlorhydrate de pyridine utilisé au stade (a) est préparé in situ par addition d'acide chlorhydrique concentré à de la pyridine et élimination de l'eau par distillation pour obtenir un résidu épais mais agitable.

3. Procédé selon la revendication 1 ou 2, **caractérisé en ce que** la réaction de déméthylation ou le stade (a) du procédé de la présente invention est également entrepris en présence de tributylamine.

4. Procédé selon la revendication 3, **caractérisé en ce que** la tributylamine est utilisée à des rapports en poids par rapport au 6-méthoxy-2-(4-méthoxyphényl)benzo[b]thiophène (II) compris entre 0,5 et 2.

5. Procédé selon la revendication 4, **caractérisé en ce que** le stade (a) est entrepris à une température comprise entre 170 et 180°C.

6. Procédé selon l'une quelconque des revendications 1-5, **caractérisé en ce que** de l'anhydride acétique est utilisé comme agent acétylant en présence de triéthylamine dans l'acétate d'éthyle.

7. Procédé selon l'une quelconque des revendications 1-6, **caractérisé en ce que** le chlorhydrate de chlorure de 4-(2-pipéridinoéthoxy)benzoyle de formule (V) utilisé au stade (c) est préparé in situ par réaction de chlorhydrate de l'acide 4- (2-pipéridinoéthoxy) benzoïque avec du chlorure de thionyle dans le chlorure de méthylène en présence de pyridine sans isoler le produit réactionnel.

8. Procédé selon l'une quelconque des revendications 1-7 **caractérisé en ce que** le stade (c) est entrepris dans le chlorure de méthylène.

9. Procédé selon la revendication 8, **caractérisé en ce que** le stade (c) est entrepris en accord avec les modalités opératives qui suivent : le 6-acétoxy-2-(4-acétoxyphényl)benzo[b]thiophène (IV) est ajouté à du chlorhydrate de chlorure de 4-(2-pipéridinoéthoxy]benzoyle non isolé de formule (V) et préparé in situ comme à la revendication 7 et le mélange ci-dessus est versé sur un mélange consistant en chlorure de méthylène et trichlorure d'aluminium.

10. Procédé selon l'une quelconque des revendications 1-9 **caractérisé en ce que** le 6-acétoxy-2-(4-acétoxyphényl)benzo[b]thiophène (IV) n'est pas isolé mais est utilisé à l'état brut à la réaction subséquente (d).

11. Procédé selon l'une quelconque des revendications 1-10 **caractérisé en ce que** le chlorhydrate de raloxifène dérivé du stade (d2) est cristallisé dans un solvant alcoolisé.

12. Procédé selon la revendication 11, **caractérisé en ce que** ledit solvant est du méthanol éventuellement en présence de HCI.

13. Procédé selon l'une quelconque des revendications 11 et 12, **caractérisé en ce que** le chlorhydrate de raloxifène est obtenu avec une pureté HPLC supérieure à 99%.

14. Procédé selon l'une quelconque des revendications 12 et 13, **caractérisé en ce qu'**une plus ample cristallisation à partir du chlorhydrate de raloxifène dans un alcol solvant est entreprise.

15. Procédé selon la revendication 14**, caractérisé en ce que** ladite cristallisation est entreprise dans le méthanol éventuellement en présence de HCI.

16. Procédé selon la revendication 15 pour la préparation de chlorhydrate de raloxifène avec une pureté HPLC supérieure à 99,7% et contenant de l'aluminium en une quantité inférieure à 5 ppm %.

17. Procédé selon la revendication 16, **caractérisé en ce qu'**il contient du N-oxyde de chlorhydrate de raloxifène en une quantité inférieure à 0,05%.

18. Procédé selon la revendication 17, **caractérisé en ce que** ladite impureté est contenue en une quantité inférieure à 0,01%.

19. Procédé selon la revendication 18, **caractérisé en ce que** le chlorhydrate de raloxifène a D(0,9) ≤ 100 µm et D(0,5) ≥ 40 µm.

20. Procédé selon la revendication 19, **caractérisé en ce qu'**il comprend un autre tamisage pour ainsi obtenir un chlorhydrate de raloxifène ayant D(0,9) entre 50 et 65 µm et D[4,3] 20 µm.
